# EUROPEAN PATENT APPLICATION

(11) **EP 4 445 739 A2**
(43) Date of publication of application: **16.10.2024**
(21) Application number: 24170126.7
(22) Date of filing: 14.04.2024
(51) Int. Cl.: A23C 9/123, A23C 9/127, A23L 33/135, C12N 1/20, C12P 1/04, C12P 39/00

(54) **METHODS FOR PRODUCTION OF BIOPRODUCTS**

(30) Priority: 14.04.2023 US 202363459262 P
(71) Applicant: Superbrewed Food, Inc., New Castle, Delaware 19720 (US)
(72) Inventor: Karpol, Alon, Tel Mond (IL); Eyal, Aharon M., Jerusalem (IL); Tracy, Bryan, Wilmington, DE (US)
(74) Representative: Harrison IP Limited

(57) **Abstract**

Provided is a method of producing a bioproduct, comprising culturing at least one target microorganism in a target growth medium comprising cells of a strictly anaerobic bacterium, the cells comprising protein at a crude protein concentration of at least 60wt% of a total dry weight of the cells, wherein at least 50% of a total number of the cells are dead cells.

## Description

### Field of the invention

The present invention, in at least some embodiments, relates to methods of production of bioproducts, and more specifically to such methods comprising culturing at least one target microorganism in a target growth medium comprising cells of a strictly anaerobic bacterium.

### Background of the invention

Methods for the production of bioproducts include growth of cells in culture, including fermentation processes as well as growth of bacterial cells and isolated animal-derived cells.

Fermentation processes rely on the action of microorganisms for the production of various products, such as food products, beverages, pharmaceutical products and the like.

Such processes are often slow and/or inefficient, requiring the use of large amounts of microorganisms and/or fermentation media.

There is thus an unmet need to provide, which are devoid of at least some of the disadvantages of the prior art.

### Summary of the invention

According to an aspect of some embodiments of the present invention, there is provided a method for producing a bioproduct, comprising culturing at least one target microorganism in a target growth medium comprising cells of a strictly anaerobic bacterium, said cells comprising protein at a crude protein concentration of at least 60wt% of a total dry weight of said cells, wherein at least 50% of a total number of said cells are dead cells.

According to a further aspect of some embodiments of the present invention, there is provided a bioproduct prepared according to the method disclosed herein.

### Detailed description of the invention

The present invention, in at least some embodiments, relates to methods for producing a bioproduct, the method comprising culturing at least one target microorganism in a target growth medium comprising cells of a strictly anaerobic bacterium.

The particulars shown herein are by way of example and for purposes of illustrative discussion of the various embodiments of the present invention only and are presented in the cause of providing what is believed to be the most useful and readily understood description of the principles and conceptual aspects of the invention. In this regard, no attempt is made to show details of the invention in more detail than is necessary for a fundamental understanding of the invention, the description making apparent to those skilled in the art how the several forms of the invention may be embodied in practice.

The present invention will now be described by reference to more detailed embodiments. This invention may, however, be embodied in different forms and should not be construed as limited to the embodiments set forth herein. Rather, these embodiments are provided so that this disclosure will be thorough and complete, and will fully convey the scope of the invention to those skilled in the art.

Unless otherwise defined, all technical and scientific terms used herein have the same meaning as commonly understood by one of ordinary skill in the art to which this invention belongs. The terminology used in the description of the invention herein is for describing particular embodiments only and is not intended to be limiting of the invention.

As used herein, the term "bioproduct" refers to a material which is obtained from or by the action of a biological source.

As used herein, the term "target microorganism" refers to a microorganism which is the major microorganism intended to be produced by the culturing process.

As used herein, the term "target growth medium" refers to a medium provided for growth therein of the target microorganism and which provides all essential nutritional requirements of the target microorganism.

As used herein, the term "strictly anaerobic bacterium" (also known as an obligate anaerobe) refers to a bacterium which are unable to grow in the presence of normal atmospheric concentrations of oxygen. According to some embodiments, the bacterium loses viability at an oxygen concentration of less than 8%, such as 8%, 7%, 6%, 5%, 4%, 3%, 2%, 1% or even 0.5%. Non-limiting examples of strictly anaerobic bacteria include bacteria of the genera Actinomyces, Bacteroides, Clostridium, Fusobacterium, Peptostreptococcus, Porphyromonas, Prevotella, Propionibacterium, and Veillonella.

As used herein, the term "cell growth medium" refers to a medium in which the cells of the strictly anaerobic bacterium is grown.

As used herein, the term "fermentation" refers to a process involving generation of energy from carbohydrates, generally under anaerobic conditions.

As used herein, the term "fermented food product" refers to a food product produced by the action of microorganisms on an edible starting material, resulting in a desirable change to the starting material.

As used herein, the term "ingestible product" refers to an product that is configured to be taken into the body by mouth, including a solid, semi-solid or liquid food product (such as a food or a beverage), a pharmaceutical product, a nutraceutical product, a probiotic and the like. As used herein, the term "food" or "food product" is intended to cover food and beverages intended for human consumption as well as animal feed.

Notwithstanding that the numerical ranges and parameters setting forth the broad scope of the invention are approximations, the numerical values set forth in the specific examples are reported as precisely as possible. Any numerical value, however, inherently contains certain errors necessarily resulting from the standard deviation found in their respective testing measurements. Every numerical range given throughout this specification will include every narrower numerical range that falls within such broader numerical range, as if such narrower numerical ranges were all expressly written herein.

As used herein, when a numerical value is preceded by the term "about", the term "about" is intended to indicate +/-10% of that value.

As used herein, the terms "comprising", "including", "having" and grammatical variants thereof are to be taken as specifying the stated features, integers, steps or components but do not preclude the addition of one or more additional features, integers, steps, components or groups thereof. These terms encompass the terms "consisting of" and "consisting essentially of".

The present inventors have surprisingly found that inclusion of strictly anaerobic bacteria in a growth medium for culturing various target microorganisms results in at least one of increased rate of production of the target microorganisms, increased yield of the target microorganisms, increased rate of production of a fermentation product produced by culturing of the target microorganisms and increased yield of the fermentation product produced by culturing of the target microorganisms.

A method for producing a bioproduct, comprising culturing at least one target microorganism in a target growth medium comprising cells of a strictly anaerobic bacterium, said cells comprising protein at a crude protein concentration of at least 60wt% of a total dry weight of said cells, wherein at least 50% of a total number of said cells are dead cells.

Additional advantages of the invention will be set forth in part in the description which follows, and in part will be obvious from the description, or may be learned by practice of the invention. It is to be understood that both the foregoing general description and the following detailed description are exemplary and explanatory only and are not restrictive of the invention, as claimed.

According to an aspect of some embodiments of the present invention, there is provided a method for producing a bioproduct, the method comprising culturing at least one target microorganism in a target growth medium comprising cells of a strictly anaerobic bacterium, the cells of the strictly anaerobic bacterium comprising protein at a crude protein concentration of at least 60wt% of a total dry weight of the cells of the strictly anaerobic bacterium, wherein at least 50% of a total number of the cells of the strictly anaerobic bacterium are dead cells.

According to some embodiments, the bioproduct is the target microorganism. According to some embodiments, the bioproduct is a fermentation product produced by the target microorganism.

According to some embodiments, the cells comprise a crude protein content of about 60wt%, about 65%, about 70wt%, about 75wt%, about 80wt%, about 85wt%, about 90% of a total dry weight of the cells.

According to some embodiments, the total number of dead cells of the strictly anaerobic bacterium is at least 50%, at least 55%, at least 60%, at least 65%, at least 70%, at least 75%, at least 80%, at least 85%, at least 90%, at least 95%, at least 99% or at least 99.9%.

According to some embodiments of the method, prior to or simultaneously with culturing the at least one target microorganism in the target growth medium comprising the cells of the strictly anaerobic bacterium, the cells of the strictly anaerobic bacterium are cultured in a cell growth medium until a predetermined cell concentration is reached; and the cells of the strictly anaerobic bacterium at the predetermined cell concentration are combined with the target microorganism in the target medium.

According to some embodiments, the predetermined cell concentration is at least about 1×10⁶ Colony Forming Units.

According to some embodiments, the composition of the cell growth medium is the same as that of the target growth medium. According to some embodiments, the composition of the cell growth medium is different from that of the target growth medium, for example with regard to components present or with regard to different concentrations of the same components.

According to some embodiments, the target microorganism and the cells of the strictly anaerobic bacterium are cultured in a single vessel.

According to some embodiments, the target microorganism and the cells of the strictly anaerobic bacterium are initially cultured in separate vessels, prior to combining.

According to some embodiments, the cells of the strictly anaerobic bacterium are cultured in the cell growth medium comprising an oxygen concentration of less than 1%; and oxygen is added to the cell growth medium to provide an oxygen concentration of between 1 and 21% prior to combining with the target microorganism.

According to some embodiments, the target microorganism is aerobic.

According to some embodiments, the target microorganism is capable of at least partially hydrolyzing the protein of the cells. According to some such embodiments, the target organism is capable of hydrolyzing at least 5% of the protein of the cells, such as about 5%, about 10%, about 15%, about 20%, about 25%, about 30%, about 35%, about 40%, about 45%, about 50%.

According to some embodiments, the target growth medium comprises an organic or inorganic source of at least one of carbon and nitrogen. According to some embodiments, the carbon source comprises lactose.

According to some such embodiments, the target growth medium further comprises at least one selected from the group consisting of an essential mineral, a vitamin and a growth factor, or combinations thereof. According to some embodiments, the essential mineral is selected from the group consisting of a macromineral (such as at least one selected from the group consisting of calcium, phosphorus, magnesium, sodium, potassium, chloride, and sulfurase, and a micromineral (such as at least one selected from the group consisting of chromium, cobalt, copper, iodine, iron, manganese, molybdenum, selenium and zinc) or a combination thereof.

According to some embodiments, the at least one target microorganism comprises at least one bacterium.

According to some embodiments, the at least one bacterium comprises a lactic acid bacterium. According to some embodiments, the lactic acid bacterium is of the family lactobacillaceae. According to some embodiments, the lactic acid bacterium is of the genus *lactobacillus.*

According to some embodiments, the strictly anaerobic bacterium is a Gram-positive bacterium. According to some embodiments, the Gram-positive bacterium is of the class *Clostridia.* According to some embodiments, the bacterium is *Clostridium tyrobutyricum.*

According to an aspect of some embodiments of the present invention, there is provided a bioproduct prepared according to the method disclosed herein.

According to some embodiments, the bioproduct is selected from the group consisting of an ingestible product, such as a food product, pharmaceutical, a nutraceutical, and a probiotic.

According to some embodiments, the food product is a fermented food product *i.e.* a food product produced by fermentation.

According to some embodiments, the fermented food product is a fermented dairy product, wherein the growth medium comprises milk.

According to some embodiments, the fermented dairy product is a yogurt.

According to some embodiments, the fermented dairy product is a cheese (such as a hard cheese, a spreadable or soft cheese or a semi-soft cheese).

According to some embodiments, the fermented food product is a fermented non-dairy food product, such as a food product selected from the group consisting of an alcoholic beverage (including wine, beer, sake and the like), vinegar, a pickled food product (such as a pickled vegetable), kimchi, tempeh, miso, kombucha and soy sauce.

According to some embodiments, the fermented product is a vitamin, such as vitamin C, vitamin B2 or vitamin B 12.

According to some embodiments, the fermented product is a biofuel.

According to some embodiments, the bioproduct is a pharmaceutical product, such as an antibiotic (including penicillin, amoxicillin, tetracycline and erythromycin), a recombinant protein (such as insulin, produced using yeast and/or bacteria), an antibody, a cytokine, a hormone, an enzyme, a blood factor, interferon, a vaccine and a tumor necrosis factor.

According to some embodiments, the bioproduct comprises a cell derived from an animal, including a human animal.

According to some embodiments, the bioproduct comprises a cell of an animal organ or organelle or a tissue culture. According to some such embodiments, the organ is the skin. According to some embodiments, the cells of the skin are suitable for use in skin grafting.

According to some embodiments, the bioproduct comprises cultured meat or seafood cells.

According to some embodiments, the target microorganism is selected from the group consisting of a bacterium, a yeast, a mold, a fungus, archaea and combinations thereof. According to some such embodiments, the target microorganism comprises two or more different strains of a microorganism or two or more different classes of microorganisms.

According to some embodiments, the bioproduct comprises the target organism.

According to some embodiments, the bioproduct comprises a metabolite of the target organism. According to some embodiments, the metabolite is selected from the group consisting of an amino acid, an alkanol, an organic acid, a protein and a peptide. According to some embodiments, the metabolite is a secondary metabolite of the target organism. According to some embodiments, the protein or peptide is a recombinant protein or recombinant peptide, respectively, such as an animal-derived protein (including a milk protein, an egg protein or a meat protein) that is produced recombinantly through yeast fermentation.

According to some embodiments, the bioproduct further comprises at least a fraction of the protein of the strict anaerobic cells.

## Claims

1. A method of producing a bioproduct, comprising culturing at least one target microorganism in a target growth medium comprising cells of a strictly anaerobic bacterium, said cells comprising protein at a crude protein concentration of at least 60wt% of a total dry weight of said cells, wherein at least 50% of a total number of said cells are dead cells.

2. The method of claim 1, wherein
(i) prior to said culturing said at least one target microorganism in said target growth medium comprising said cells, said cells are cultured in a cell growth medium until a predetermined cell concentration is reached; and
(ii) said cells at said predetermined cell concentration are combined with said target microorganism in said target medium.

3. The method of claim 1 or claim 2, wherein a composition of said cell growth medium is different from a composition of said target medium.

4. The method of any one of claims 1 to 3, wherein said target microorganism and said cells are cultured in a single vessel.

5. The method of claim 2, wherein said cells are cultured in said cell growth medium comprising an oxygen concentration of less than 1%; and oxygen is added to said cell growth medium to provide an oxygen concentration of between 1 and 21% prior to or simultaneously with combining with said target microorganism.

6. The method of any one of claims 1 to 5, wherein said target microorganism is aerobic.

7. The method of any one of claims 1 to 6, wherein said target microorganism is capable of at least partially hydrolyzing said protein of said cells.

8. The method of any one of claims 1 to 7, wherein said target growth medium comprises at least one carbon source.

9. The method of claim 8, wherein said carbon source comprises lactose.

10. The method of any one of claims 1 to 9, wherein said at least one target microorganism comprises at least one bacterium comprising a lactic acid bacterium.

11. The method of any one of claims 1 to 10, wherein said strictly anaerobic bacterium is a Gram-positive bacterium.

12. A bioproduct prepared according to the method of any one of claims 1 to 11.

13. The bioproduct of claim 12, selected from the group consisting of a food product, a beverage, a pharmaceutical, a nutraceutical, and a probiotic.

14. The bioproduct of claim 13, wherein said food product is a fermented food product.

15. The bioproduct of claim 14, wherein said fermented food product is selected from the group consisting of a fermented dairy product, wherein said growth medium comprises milk; and a fermented non-dairy food product.
